# EUROPEAN PATENT APPLICATION

(11) **EP 4 287 200 A1**
(43) Date of publication of application: **06.12.2023**
(21) Application number: 22184967.2
(22) Date of filing: 14.07.2022
(51) Int. Cl.: G16H 30/20, G16H 30/40

(54) **SYNCHRONIZING AUDIOVISUAL DATA AND MEDICAL DATA**

(30) Priority: 01.06.2022 US 202263347679 P
(71) Applicant: Koninklijke Philips N.V., 5656 AG Eindhoven (NL)
(72) Inventor: ERKAMP, Ramon Quido, Eindhoven (NL); KYNE, Sean, Eindhoven (NL); SINHA, Ayushi, 5656AG Eindhoven (NL); FOTOUHI, Javad, 5656AG Eindhoven (NL); SALEHI, Leili, Eindhoven (NL); PAI RAIKAR, Vipul Shrihari, Eindhoven (NL)
(74) Representative: Philips Intellectual Property & Standards

(57) **Abstract**

The present invention relates to a computer-implemented method of synchronizing audiovisual data and medical data, the method comprising: receiving the audio-visual data (40) recorded by a first device (20), the audio-visual data including an audio channel and a video channel simultaneously capturing a medical procedure performed in a medical environment, receiving the medical data (30) recorded by a second device, the medical data capturing physiological parameters of a patient during the medical procedure; classifying, using one or more machine learning algorithms, one or more sounds from the audio channel of the audio-visual data as being produced by equipment in the medical environment; and synchronizing the audio-visual data with the medical data based on a time of occurrence of the one or more sounds.

## Description

### FIELD

The present invention relates to a computer-implemented method of synchronizing audiovisual data and medical data, a computer-implemented method of training a machine learning algorithm, and a transitory or non-transitory computer readable medium. More specifically, the medical data may capture physiological parameters of a patient during a medical procedure and the audiovisual data may capture the medical procedure.

### BACKGROUND

Medical data, such as medical images, can be captured for various reasons, for example to record a patient's condition or physiological parameters during a medical procedure. For example, during an image guided intervention, medical images such as X-ray images may be used to monitor the progress of the procedure and to view the patient's internals, providing the ability to observe devices such as guidewires, catheters and stents inside the patient in real-time. Sometimes, medical professionals will also record a video of the medical procedure on a separate device, e.g. a mobile device such as a cell phone. It is sometimes desired to synchronize the medical data and the recorded video.

It is an object of the present invention to improve on the prior art.

### SUMMARY

According to a first aspect of the present invention, there is provided a computer-implemented method of synchronizing audiovisual data and medical data, the method comprising: receiving the audio-visual data recorded by a first device, the audio-visual data including an audio channel and a video channel simultaneously capturing a medical procedure performed in a medical environment, receiving the medical data recorded by a second device; classifying, using one or more machine learning algorithms, one or more sounds from the audio channel of the audio-visual data as being produced by equipment in the medical environment; and synchronizing the audio-visual data with the medical data based on a time of occurrence of the one or more sounds.

In this way, it is easy to synchronize the audio-visual data and the medical data, for example for the purpose of producing a composite video, because this method does not rely on sound features like humans speaking specific terms, which is distracting for the medical personnel. Instead, this method provides a passive means for synchronizing the respective data modalities.

In an embodiment, the equipment may be a medical imaging system comprising the second device, and the medical data may include medical images, such as X-ray images.

In further examples, the method further comprises logging, in an event log, one or more events associated with the medical equipment, and wherein the synchronizing may comprise temporally matching the one or more sounds with the respective one or more events from the event log. This may provide an advantage because it enables passive synchronization of features existing in the medical data at different phase and equipment used during those phases. In particular, events may be logged while medical images are captured by the second device. For example, when the second device is a C-arm X-ray imaging device as used during certain medical interventions in a Cath lab, the temporal matching may involve logged cathlab events.

In certain examples, the one or more sounds produced by the equipment includes a sound signature produced by a speaker system controlled by the second device, the sound signature indicating a system clock time of the second device, wherein the synchronizing the audio-visual data comprises temporally matching the one or more sounds with one or more time stamps indicative of the system clock time.

In this way, accurate synchronization is provided by actively projecting the time signature from the second device to the first device.

In an embodiment, producing the sound signature may comprise: producing a first sound pattern at a first frequency; producing a second sound pattern at a second frequency, wherein the first frequency and the second frequency may be above a human audible frequency range, and a difference between the first frequency and the second frequency may be within the human audible frequency range. In this way, the first and second frequencies are undetectable by humans and so the medical personnel are not distracted during the medical procedure, yet the difference frequency is within a recording frequency range of a microphone

In an embodiment, the computer-implemented method may further comprise: detecting, using one or more machine learning algorithms, a display of the second device in the video channel of the audio-visual data; identifying, using the one or more machine learning algorithms, one or more display features on the display of the second device; and wherein the synchronizing the audio-visual data with the medical data further comprises matching a time of occurrence of the one of more display features in the audio-visual data with a time of occurrence of displaying the one or more features on the display by the second device. In this way, accuracy of the synchronization is improved by matching displayed features on the second device with features recorded by the first device. In this way, the sounds detecting the equipment being used may be fine-tuned using the image feature detection.

In an embodiment, the one or more display features may comprise a system clock of the second device. System clocks may be accurate to the degree of granularity of time measurements of the displayed clock. Where the time is provided in seconds, this may help fine tune the synchronization

In an embodiment, the one or more display features may comprise the medical data. When display features change, their timing can be detected almost instantaneously, again fine-tuning synchronization.

According to a further aspect of the present invention, there is provided a computer-implemented method of generating a composite video. The method comprises: synchronizing audiovisual data and medical data as described above; detecting one or more video features from the medical procedure in the video channel of the audio-visual data; and generating the composite video by identifying a portion of the synchronized audio-visual data and medical data for display based on a time of occurrence of the or each video feature.

In an embodiment, the one or more video features may comprise first and second video features, and wherein the generating the composite video may comprise: identifying a first time of occurrence of the first video feature in the video channel; identifying a second time of occurrence of the second video feature in the video channel; and identifying the portion of the composite video as being between the first time of occurrence and the second time of occurrence.

In an embodiment, the computer-implemented method may further comprise displaying the portion of the composite video on a display of the first device.

In an embodiment, the first video feature may comprise recording start and the second video features comprises recording end. Each recording must have a start and an end point and so this is a reliable way to provide the first and second display features. Also, this approach is passive since no further features need to be actively detected.

In an embodiment, the generating the composite video data may comprise augmenting the video channel with the medical data.

In an embodiment, the one or more display features may comprise the medical data, wherein the augmenting the video channel with the medical data may comprise replacing medical data in the video channel identified as the one or more display features on the display of the second device, with the medical data recorded by the second device. In this way, the resolution of the medical data captured by the second device may higher than the medical data displayed on the display of the second device and recorded by the first device

In an embodiment, the medical data may be a medical image.

According to a further aspect of the invention, there is provided a computer-implemented method of training one or more machine learning algorithms, the method comprising: providing an audio channel from audio-visual data and a label identifying one or more sounds in the audio channel of equipment used in a medical environment; and training the one or more machine learning algorithms using supervised learning based on the label to classify the one or more sounds in the audio channel as being associated with the equipment.

According to a further aspect of the invention, there is provided a transitory, or non-transitory computer readable medium, having instructions stored thereon that, when executed by a processor, cause the processor to perform the method of any preceding claim.

These and other aspects of the invention will be apparent from and elucidated with reference to the embodiment(s) described hereinafter.

### BRIEF DESCRIPTION OF DRAWINGS

The embodiments of the inventions described herein are best understood with reference to the accompanying drawings, in which:
Figure 1 shows a perspective view of a medical environment including medical equipment and a medical imaging device;
Figure 2 shows a perspective view of a mobile device used for capturing an audio-visual recording of a medical procedure carried out in the medical environment form Figure 1;
Figure 3 shows a block diagram of a document management system for managing the medical data and the audio-visual data according to an embodiment of the invention;
Figure 4 shows a schematic diagram illustrating a method of synchronizing medical data and the audio-visual data according to an embodiment of the invention;
Figure 5 shows a schematic diagram illustrating a method of identifying sounds from an audio channel of the audio-visual data according to an embodiment of the invention for use in the method of synchronizing from Figure 4;
Figure 6 shows before and after perspective views of a mobile device respectively displaying a video and a composite video including the medical data and the audio-visual data according to an embodiment of the invention;
Figure 7 shows before and after perspective views of a mobile device respectively displaying a video and a composite video including the medical data and the audio-visual data according to an embodiment of the invention;
Figure 8 shows a flow chart of a method of synchronizing audio-visual data and medical data according to an embodiment of the invention; and
Figure 9 shows a flow chart of a method of training one or more machine learning algorithms according to an embodiment of the invention.

### DESCRIPTION OF EMBODIMENTS

The methods described herein may be computer-implemented methods. In particular, the methods may include at least a computer-implemented method of generating a composite video and a computer-implemented method of training one or more machine learning algorithms. The computer which implements the methods may include a storage, e.g. memory, and a processor. The computer may be a hardware computer and thus the storage and the processor may respectively be a hardware storage and a hardware processor.

The computer-implemented methods may be provided as instructions stored on a transitory, or non-transitory, computer-readable medium. The computer-readable medium may be stored in the storage of the computer. When the instructions are executed by the processor, the instructions cause the processor to perform any of the method steps descried herein.

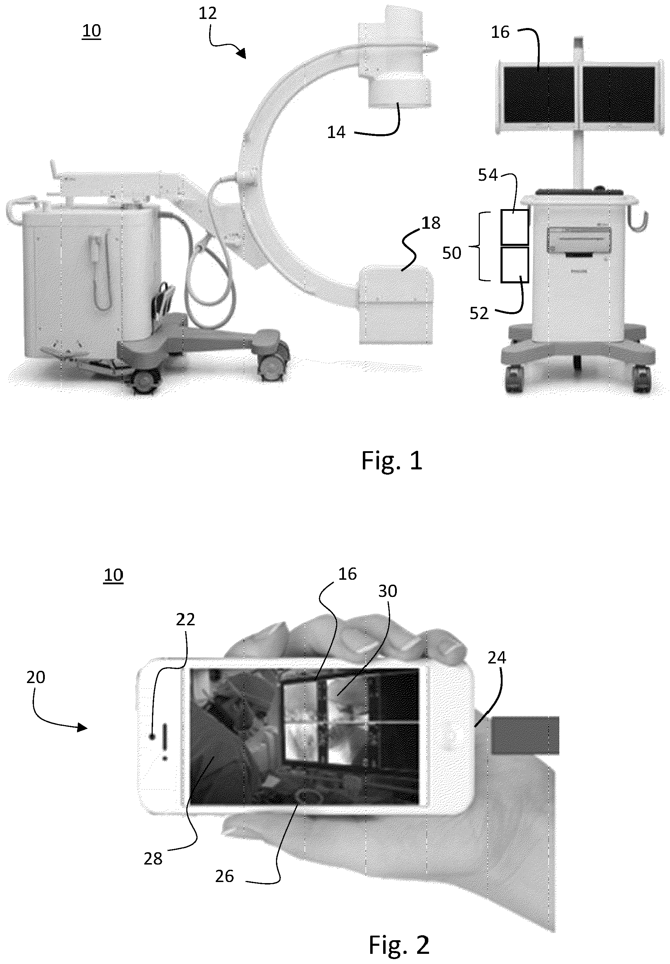

With reference to Figure 1, a medical procedure is carried out in a medical environment 10, e.g. an operating theatre. The medical environment 10 includes equipment 12. The equipment may include medical equipment for performing the medical procedure, e.g. a table, a drip, a catheter, a scalpel, etc. In addition, the equipment may include a medical data capturing device (otherwise called herein a second device) 12. In Figure 1, the medical data capturing device 12 is a C-arm X-ray imaging system. In other instances, different medical data capturing devices may be used where the medical procedure requires it. The medical data capturing device 12 may include a sensor 14 and a display 16. The sensor 14 may be configured to capture physiological parameters of a patient during the medical procedure. In the case of C-arm, an emitter or source 18 may also be provided. The emitter 18 may emit X-ray signals, and the sensor 14 may detect X-rays. The medical data generated by the C-arm may thus be a medical image. The medical image may be an X-ray. In other embodiments, the medical image may be, for example, a computed tomography (CT) scan image, an ultrasound scan image, a magnetic resonance imaging scan, etc. The display 16 may display the medical image in real time during the medical procedure to provide guidance to the medical personnel.
With reference to Figure 2, also within the medical environment 10 is an audio-visual recorder 20 for recording audio-visual data. The audio-visual recorder 20 may be a mobile device and may otherwise be called herein a first device. The mobile device may be a smartphone or tablet. The audio-visual recorder 20 may also be part of another system such as an augmented reality (AR) headset or a head mountable camera.

During the medical procedure, a user, e.g. a medical professional, may wish to record a phase of the medical procedure. The recording may be used for training, for example. The user may record the phase of the medical procedure 28 using the first device 20. The first device 20 may include a camera 22, a microphone 24, and a display 26. The display 26 may be in the form of a touch screen.

The camera 22 may be configured to capture video data of its field of view. In this context, the field of view includes the medical environment 10. The microphone 24 may be configured to capture audio data from within the vicinity of the first device 20. The video data and the audio data may be synchronized using the internal clock of the first device 20. The synchronized audio and visual data may be called audio-visual data. In this way, the audio-visual data may include an audio channel, representing the audio data, and a video channel, representing the video data. The audio channel and the video channel have captured simultaneously the medical procedure, or at least a phase of the medical procedure, in the medical environment. As will be described in more detail below, the display 16 of the second device 12 may also be recorded in the video channel of the audio-visual data. The display 16 may be displaying medical data (e.g. a medical image) 30 captured by the second device 12.

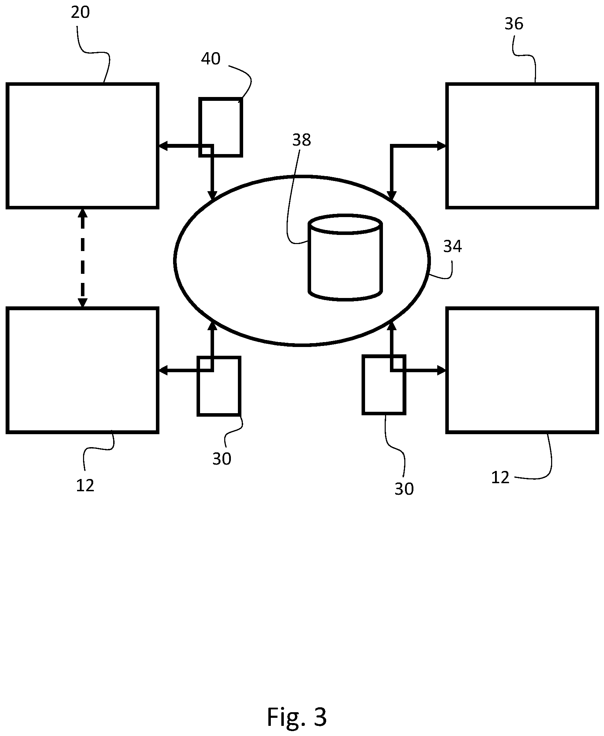

With reference to Figure 3, a database system may be provided to manage and store medical data captured by one or more second devices 12. The database system may also manage and store audio-visual data captured by one or more first devices 20.

The database system may include a server 34. The first device 20 and the second device 12 may be communicatively linked to the server 34. The communicative link may be provided as a wireless link, e.g. Bluetooth or wi-fi, or may be a physical connection, e.g. via a cable or wire. In some embodiments, the first and second devices 20, 12, may be connected together directly, i.e. in addition to being indirectly connected via the server 34. The direct connection may be provided wirelessly, e.g. Bluetooth or wi-fi, or may be a physical connection, e.g. via a cable or wire.

One or more user interfaces 36 may also be provided and communicatively linked to the server 34 to access, view, and edit, for example, any data stored by the server 34. In this way, the server 34 may be connected to a storage medium which stores any audio-visual data 40 and medical data 30 received from respective first 20 and second 12 devices.

The database system may be a Picture Archiving and Communication System (PACS). The medical data may conform to a standard. The standard may be Digital Imaging and Communication in Medicine (DICOM).

According to one or more embodiments of the invention, a method is provided for synchronizing the audio-visual data and the medical data. The method may be performed by the document management system, by the first device 20, by the second device 12, or by another device, e.g. the server 34.

The method starts by receiving the audio-visual data 40 recorded by the first device 20 and receiving the medical data 30 recorded by the second device 12. Next, the method comprises classifying, using a machine learning algorithm, one or more sounds from the audio channel of the audiovisual data 40 as being produced by equipment in the medical environment 10.

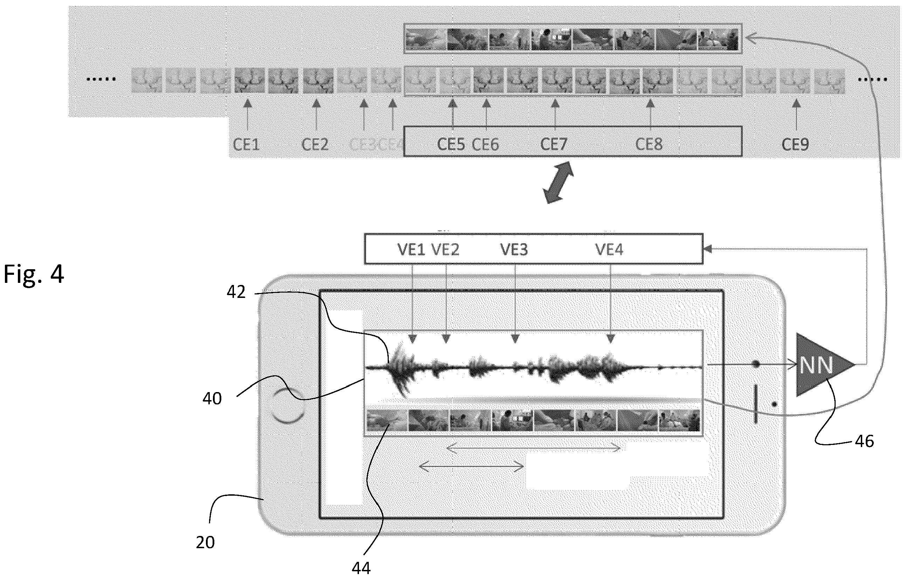

With reference to Figure 4, the first device 20 captures the audio-visual data 40 of the phase of the medical procedure. The audio-visual data 40 includes the audio channel 42 and the video channel 44. Whilst the audio-visual data 40 is shown on the display 26 of the first device 20, this is purely shown diagrammatically and only for illustrative purposes. In practice, the display 26 will not display visibly the audio-visual data in terms of trace/waveform data and image frames.

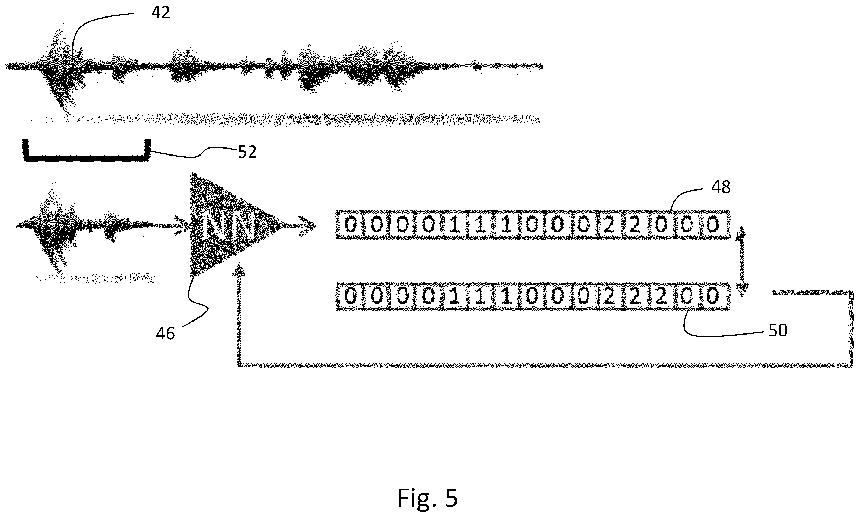

With reference to Figure 5, the audio channel 42 is input to a machine learning algorithm 46. The machine learning algorithm 46 may be a supervised machine learning algorithm. The supervised machine learning algorithm may be trained by providing an audio channel from audio-visual data and a label identifying one or more sounds in the audio channel of equipment used in a medical environment. The machine learning algorithm may be trained using supervised learning based on the label to classify the one or more sounds in the audio channel as being associated with the equipment.

The machine learning algorithm may be a neural network. In this embodiment, the neural network may be a recurrent neural network. In other embodiments, the neural network may be a convolutional neural network or a transformer network.

The training may include forward propagation and back propagation. In forward propagation, samples 52 from the audio channel of training data are input to the neural network. The samples 52 may be taken periodically. The samples 52 may be of substantially equal duration. Each sample 52 is passed through the neural network which outputs a value using an output layer. The output layer may include a softmax layer. The softmax layer may include a plurality of nodes each representing the probability that the sample of audio data has been produced by a particular event. The particular event may be a sound produced by medical equipment in the medical environment.

The neural network generates an output vector 48 including a plurality of values. Each value corresponds to a classification of a sample 52. In other words, when the neural network decides that the sample corresponds, or has the highest probability of corresponding, to a particular source, a value for that event is provided. For example, a value of one may correspond to a first source.

For example, output zero may correspond to background noise, output one may correspond to a piece of medical equipment moving, e.g. a table being lowered, output two may correspond to a piece of medical equipment, e.g. the second device 12 being moved, etc.

A loss or error is calculated between the output vector 48 and a ground truth vector 50 using a loss function. Back propagation is used to optimize the hyperparameters, e.g. the weights within the layers, of the neural network based on the loss function. The loss function may be a least absolute deviations (LI) loss function or a least square errors (L2) loss function.

As discussed above, the task of the neural network is to detect and classify the characteristic audio events (one or more sounds) in the presence of other background noise while accounting for acoustic changes in the recorded audio due to position relative to the C-arm and acoustic properties of the recording device, room, etc. A method for generating training data for such a network, is to collect audio data that is synchronized to a system event log and associated with the medical data at a variety of positions and with a variety of recording devices in the presence of a variety of background noises, and use the system event log to label the collected audio data. Alternatively, a known series of events in the medical data could be triggered and a recording obtained. These recordings may be made with various recording systems, with various background sounds, from various positions in the medical environment relative to medical data capturing equipment, etc. The neural network is trained to identify from these recordings relevant sounds from equipment that correspond to recorded events in the system logs.

With further reference to Figure 4, in this embodiment, four sounds are identified by the neural network. The present invention is not limited to there being specifically four sounds. Those four sounds are indicated in Figure 4 as VE1, VE2, VE3, and VE4. VE may be an acronym for video event. The term "video event" is intended to mean audio-visual event since the actual event is a sound in the audio channel.

The first video event, VE1, or first sound, may be the sound of the C-arm starting to move. The second video event, VE2, or second sound, may occur after the first video event, VE1. The second video event, VE2, may be the sound of a pedal of the C-arm machine being pressed. The third video event VE3, or third sound, may be the sound of the C-arm stopping moving, or when the brake of the C-arm engages. The fourth video event VE4, or fourth sound, may be the sound of the pedal being released. In this way, the method comprises classifying the sounds as being produced by medical equipment in the medical environment during a phase of the medical procedure.

When a sequence of events like these are detected, the timing between these events creates a very specific pattern.

These sounds producing events may occur in a Catheterization lab or Cath lab equipped with a first and second device such that their clocks are synchronized. In this way, any pattern of sound producing events detected in the audio stream of the first device 20 may be associated with a matching event pattern in the event log of the second device 12, allowing synchronization of the two clocks.

For example, the cathlab events may be described using the notation CE1, CE2, ..., CEn. In the embodiment shown in Figure 4, there are nine cathlab events, CE1 to CE9. A first cathlab event, CE1, and a second cathlab event CE2, may be due to a patient table being moved up. Third and fourth cathlab events, CE3, CE4, may be due to the table being translated towards the C-arm. Fifth to eighth cathlab events, CE5 to CE8, may respectively correspond to the first to fourth video events, VE1 to VE4. The ninth cathlab event CE9 may be an arbitrary event, for example the table being adjusted or the C-arm pedal being pressed to acquire a subsequent image.

It is to be noted that the first to fourth video events VE1 to VE4 and the fifth to eighth cathlab events, CE5 to CE8, occur during a phase of the medical procedure. The phase of the medical procedure may be a particularly important phase. For instance, it may be assumed that a physician or other Cath lab staff would record a phase of the medical procedure on the first device 20 because it is a particularly important or significant phase.

In this way, the method comprises synchronizing the audio-visual data with the medical data based on a time of occurrence of the sounds. In particular, the synchronizing is enabled by temporally matching the occurrences of the sounds from the audio channel, VE1 to VE4, with the matching cathlab events, CE5 to CE8.

It will be appreciated that the equipment may be the medical imaging system comprising the second device, and the medical data includes medical images. The method may further comprise logging, in an event log, one or more events performed by the medical imaging system when the medical images are captured by the second device. The synchronizing may comprise temporally matching the one or more sounds with the respective one or more events from the event log.

Returning briefly to Figure 1, in a further embodiment, the second device 12 may further comprise a speaker 50. The speaker 50 is synchronized with the medical data that is recorded by the second device 12 because it is connected to the same system clock. In other words, the speaker 50 is controlled by the second device 12. The speaker 50 is configured to generate a sound. The sound may be in the form of a sound signature. The sound signature may indicate the system clock of the second device. For example, the temporal pattern of pulses is indicative of the time of the system clock (for example a short pulse indicates a 0 and a longer pulse a 1, and a binary sequence is transmitted encoding the clock time).

The microphone 24 of the first device 20 may detect the sound signature. The audiovisual data recorded at the same time as the sound signature is recorded and may be tagged with a time stamp. In this way, the synchronizing the audio-visual data comprises aligning the timestamps in the audiovisual data with matching timestamps in the medical imaging system. Some interpolation may be required if time shift between clocks is a fraction of clock resolution as will be appreciated by one skilled in the field.

In the same or another embodiment, the sound signature may comprise a first sound pattern and a second sound pattern. The speaker 50 may be configured to generate the first sound pattern at a first frequency and the second sound pattern at a second frequency. The first frequency may be 40kHz and the second frequency may be 50kHz. Those specific frequency values may be different, although it is preferable for the first and second frequencies to be above the audible limit for human hearing. In addition, it is preferable for the first and second frequencies to be different to one another. In this way, the nonlinearity in the sound recording hardware associated with the microphone 24 can be harnessed. This is because the nonlinearities in a mechanical membrane of the microphone 24 and initial amplifier stage will generate sum and difference frequency components. For the example of using 40kHz and 50kHz frequencies, the difference frequency is 10kHz. 10kHz is within the microphone recording frequency range for most devices that are specifically configured to record human audible sounds, e.g. microphones of mobile devices such as smartphones. Thus, even though the first and second frequencies are above the audible range of the human ear, they will lead to an audible 10kHz signal being recorded by the first device 20. In this way, the sound signature may be transmitted without distracting the medical personnel within the medical environment 10. Alternatively, the speaker 50 may include a first speaker 52 and a second speaker 54. The first speaker 52 may be configured to generate the first sound pattern at a first frequency. The second speaker 54 may be configured to generate the second sound pattern at a second frequency.

In addition to the above synchronization methods, further embodiments may include additional synchronization methods.

For example, the method may further comprise detecting, by the first device 20, using a machine learning algorithm, the display 16 of the second device 12 in the video channel of the audiovisual data. In addition, the machine learning algorithm may be used to identify one or more display features on the display 16. The machine learning algorithm may comprise a neural network. The neural network may be a convolutional neural network. The convolutional neural network may be trained to classify the display as a display, and also trained to classify the one or more display features. The classifications of the display features may include classifying the display features as a system clock of the second device 12. In addition, or as an alternative, the machine learning algorithm may be trained to classify the display features as the medical data 30 that is being displayed on the display 16.

In this way, synchronizing the audio-visual data with the medical data may further comprise matching a time of occurrence of the one or more display features with a time of occurrence of displaying the one or more features on the display by the second device.

With reference to Figure 3, regardless as to the specific synchronizing method used, the synchronized audio-visual data and medical data may be stored in the storage medium.
In addition, the method may comprise generating a composite video by identifying a portion of the synchronized audio-visual data and medical data for display based on a time of occurrence of the or each video feature.

For example, the video features may include a first video feature and a second video feature. A first time of occurrence of the first video feature may be identified in the video channel. A second time of occurrence of the second video feature may be identified in the video channel. The portion of the synchronized audio-visual and medical data may be identified as being between the first and second times of occurrence. In this way, the composite video may be generated to include the synchronized audio-visual data and medical data between the first and second times of occurrence. The composite video may be displayed on a display.

With reference to Figure 1, the display may be the display 26 of the first device 20, the display 16 of the second device 12, or another display.

In one or more embodiments, the first video feature may be recording start. In other words, when a user of the first device 20 starts recording the phase of the medical procedure, the time of the recoding start is identified. The second video feature may be recording end. In other words, when a user of the first device 20 stops recording the phase of the medical procedure, the time of the recording end is identified. It may be assumed that what the user is recording is an important phase of the medical procedure. Using recording start and end negates the need for further video features to be identified. In other words, using recording start and recording end is a passive way of identifying an important phase of the medical procedure.

In addition, in one or more embodiments, any data (e.g. medical data) that is not identified as being between the first and second video features, can be discarded, or deleted. In this way, the storage space (Figure 3) may be freed up.

The data captured during the time of the recording may also be used as training data to train the various machine learning algorithms described herein.

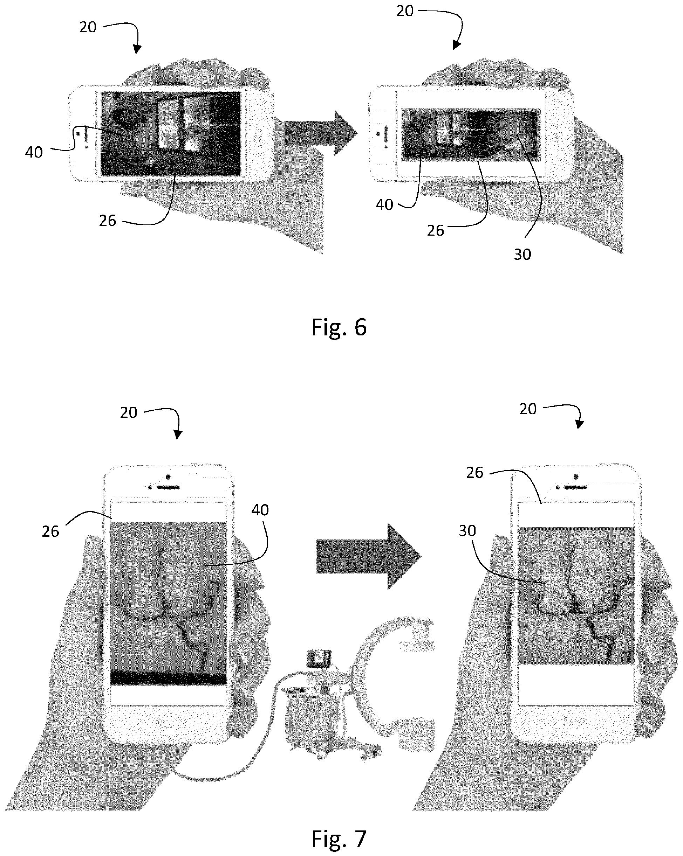

With reference to Figure 6, in one or more embodiments, the composite video may be constructed by augmenting the video channel of the audio-visual data with the medical image. For example, the first device 20 on the left of Figure 6 shows the raw video channel being displayed on the display 26. The first device 20 on the right of Figure 6 shows the video channel of the audio-visual data 40 augmented with the medical image 30.

With reference to Figure 7, in one or more embodiments, the composite video may be constructed by replacing medical data in the video channel of the audio-visual data with the medical image recorded directly by the second device 12. For example, the first device 20 on the left of the Figure 7 shows the video channel of the audio-visual data 40 being displayed on the display 26. The video channel includes the medical image that the first device 20 has captured by focusing on the display of the second device 16 (Figure 1). The first device 20 on the right of Figure 6 shows the medical image in the video channel of the audio-visual data replaced with the medical image 30 recorded by the second device 12. Replacement of the medical image with the medical image captured by the second device 12 is advantageous because it has higher resolution than the video captured by the first device 20, which also suffers from various image artifacts that result from capturing data shown on a screen (Moire effect, aliasing, specular reflection off the screen, etc.).

In one or more embodiments, one may wish to label the composite video based on speech recognition from the audio channel of the audio-visual data.

In such an embodiment, one could look for key words to determine stage of the procedure or events of interest. These labels could be attached to the stored medical data for more efficient review or put as caption in the video content when generating training material. Some example phrases to listen for include:
"Prep the groin", indicates we are close to starting the invasive part of the procedure
"close it up", indicates end of invasive part of procedure
"let's do a run", may indicate contrast is about to be injected or fluoroscopy imaging is about to be acquired
"hold your breath", significant medical data is about to be collected over the next few seconds (e.g., fluoroscopy sequence with breath hold)
"You can breathe", done collecting data for now
"give heparin", important to document administration of medications

If medical data is currently manually labelled, a deep learning system could over time be trained to associate labels with phrases from speech recognition on the audio track and start suggesting labels to gradually automate the process.

In further embodiments, when including medical data in the documentation, one could automatically remove personally identifiable information such as patient name and other details. This can be achieved by removing identifiable information from DICOM headers. If this information is present as overlay in medical image data or in audio-video data, this may be achieve by using any method that is able to detect personally identifiable information in the medical image data, (e.g. a patient's name, national health number, date of birth, etc. overlaid on the medical image data which may be blurred), or in the audio-video data (e.g., patient's face which may be blurred, or Cath lab staff mentioning patient age in the video channel which may be cut out of the audio channel).

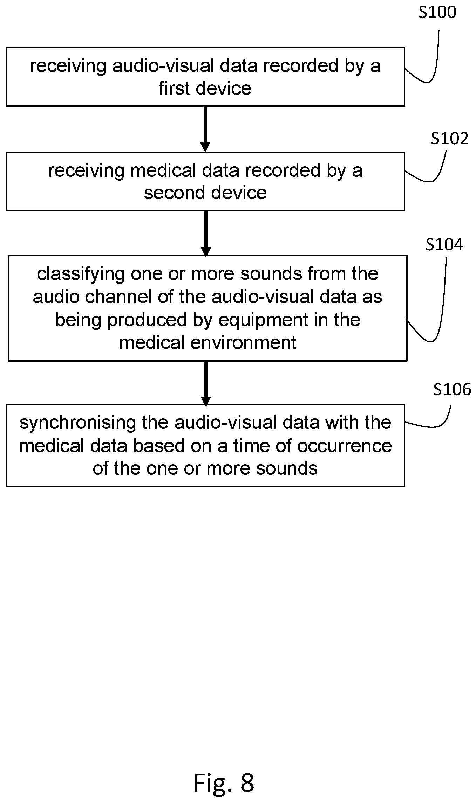

With reference to Figure 8, whilst the foregoing description describes the various embodiments in detail, some embodiments can be summarized as relating to a computer-implemented method of synchronizing audio-visual data and medical data. The method comprise various steps. At step S100, the method comprises receiving audio-visual data recorded by a first device, the audio-visual data including an audio channel and a video channel simultaneously capturing a medical procedure performed in a medical environment. At step S102, the method comprises receiving medical data recorded by a second device. At step S104, the method comprises classifying, using one or more machine learning algorithms, one or more sounds from the audio channel of the audio-visual data as being produced by equipment in the medical environment. At step S106, the method comprises synchronizing the audiovisual data with the medical data based on a time of occurrence of the one or more sounds.

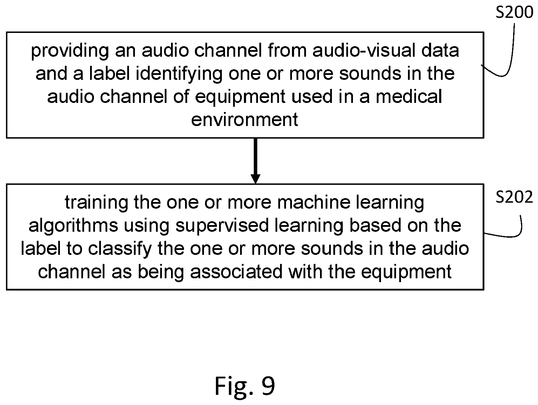

With reference to Figure 9, whilst the foregoing description describes various embodiments in detail, some embodiments can be summarized as relating to a computer-implemented method of training one or more machine learning algorithms using various steps. At step S200, the method comprises providing an audio channel from audio-visual data and a label identifying one or more sounds in the audio channel of equipment used in a medical environment. At step S202, the method comprises training the one or more machine learning algorithms using supervised learning based on the label to classify the one or more sounds in the audio channel as being associated with the equipment.

While the invention has been illustrated and described in detail in the drawings and foregoing description, such illustration and description are to be considered illustrative or exemplary and not restrictive; the invention is not limited to the disclosed embodiments. For example, it is possible to operate the invention in an embodiment wherein more than two capturing devices are used, and using more than two modalities (e.g. more than audio-visual data and medical images).

If there are many information sources, one may want to show a different subset of sources at different times in the procedure, depending on what is going on. When generating training material, a user interface could be used: First, a video is created with all information panes and a check box on each. The user can then check/uncheck the desired panes in time. Now the system generates a new video (e.g. a composite video) showing only desired information panes at the different times. AI methods could be used to predict what the desired panes are as a function of time (e.g., procedure cards).

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method of synchronizing audiovisual data and medical data in a medical procedure, the method comprising:
receiving (S100) the audio-visual data (40) recorded by a first device (20), the audiovisual data including an audio channel and a video channel simultaneously capturing the medical procedure performed in a medical environment (10),
receiving (S102) the medical data (30) recorded by a second device (12);
classifying (S104), using one or more machine learning algorithms, one or more sounds from the audio channel of the audio-visual data as being produced by equipment in the medical environment; and
synchronizing (S 106) the audio-visual data with the medical data based on a time of occurrence of the one or more sounds.

2. The computer-implemented method of Claim 1,
wherein the equipment is a medical imaging system comprising the second device, and the medical data includes medical images.

3. The computer-implemented method of Claim 1 or 2,
wherein the method further comprises logging, in an event log, one or more events associated with the medical equipment, and
wherein the synchronizing (S106) comprises temporally matching the one or more sounds with the respective one or more events from the event log.

4. The computer-implemented method of any preceding claim,
wherein the one or more sounds produced by the equipment includes a sound signature produced by a speaker system (50) controlled by the second device (12), the sound signature indicating a system clock time of the second device (12),
wherein the synchronizing the audio-visual data (40) comprises temporally matching the one or more sounds with one or more time stamps indicative of the system clock time.

5. The computer-implemented method of any preceding claim, further comprising:
detecting, using one or more machine learning algorithms, a display (16) of the second device (12) in the video channel of the audio-visual data (40);
identifying, using the one or more machine learning algorithms, one or more display features on the display (16) of the second device; and
wherein the synchronizing the audio-visual data (40) with the medical data further comprises matching a time of occurrence of the one of more display features in the audio-visual data with a time of occurrence of displaying the one or more features on the display by the second device (12).

6. The computer-implemented method of Claim 5, wherein the one or more display features comprises a system clock of the second device (12).

7. The computer-implemented method of Claim 5 or Claim 6, wherein the one or more display features comprises the medical data (30).

8. A computer-implemented method of generating a composite video, the method comprising:
synchronizing audiovisual data and medical data according to any preceding claim;
detecting one or more video features from the medical procedure in the video channel of the audio-visual data; and
generating the composite video by identifying a portion of the synchronized audio-visual data and medical data for display based on a time of occurrence of the or each video feature.

9. The computer-implemented method of Claim 8, wherein the one or more video features comprises first and second video features, and wherein the generating the composite video comprises:
identifying a first time of occurrence of the first video feature in the video channel;
identifying a second time of occurrence of the second video feature in the video channel; and
identifying the portion of the composite video as being between the first time of occurrence and the second time of occurrence.

10. The computer-implemented method of claim 9, wherein the first video feature comprises recording start and the second video features comprises recording end.

11. The computer-implemented method of any of claims 8 to 10, wherein the generating the composite video data comprises augmenting the video channel with the medical data (30).

12. The computer-implemented method of Claim 11, wherein the one or more display features comprises the medical data (30), wherein the augmenting the video channel with the medical data (30) comprises replacing medical data in the video channel identified as the one or more display features on the display of the second device (12), with the medical data (30) recorded by the second device (12).

13. A computer-implemented method of training one or more machine learning algorithms, the method comprising:
providing (S200) an audio channel from audio-visual data (40) and a label identifying one or more sounds in the audio channel of equipment used in a medical environment (10); and
training (S202) the one or more machine learning algorithms using supervised learning based on the label to classify the one or more sounds in the audio channel as being associated with the equipment.

14. A transitory, or non-transitory computer readable medium, having instructions stored thereon that, when executed by a processor, cause the processor to perform the method of any preceding claim.
